# EUROPEAN PATENT APPLICATION

(11) **EP 4 726 727 A1**
(43) Date of publication of application: **15.04.2026**
(21) Application number: 24832593.8
(22) Date of filing: 28.06.2024
(51) Int. Cl.: G16H 15/00, G06F 40/20, G06T 11/00

(54) **METHOD, COMPUTER DEVICE AND NON-TRANSITORY COMPUTER-READABLE RECORDING MEDIUM FOR GENERATING DIAGRAM OF TOOTH POSITIONS, AND METHOD, COMPUTER DEVICE AND NON-TRANSITORY COMPUTER-READABLE RECORDING MEDIUM FOR GENERATING TREATMENT PLAN**

(30) Priority: 30.06.2023 US 202363511183 P
(71) Applicant: Dentall (Singapore) Pte. Ltd., Singapore 068913 (SG)
(72) Inventor: WANG, Ting-yu, Taipei City, 100032 (TW)
(74) Representative: Rapisardi, Mariacristina
(86) International application number: PCT/SG2024/050428
(87) International publication number: WO 2025/005887

(57) **Abstract**

The present application provides a method for generating a dental chart. The method includes: inputting a medical record data into a first large language model; analyzing the medical record data by the first large language model and then generating and outputting an intermediate file corresponding to the medical record data; inputting the intermediate file into a image generating model; and analyzing the intermediate file by the image generating model and then generating and outputting the dental chart corresponding to the intermediate file. The intermediate file has at least one dental position and at least one symptom information corresponding to the at least one dental position. Thereby, the method can convert a patient's medical record data into a corresponding dental chart, in order to directly know the patient's condition. In addition, a computing device and a non-transitory computer-readable recording medium for generating the dental chart are also provided.

## Description

### BACKGROUND OF THE INVENTION

### 1. Field of the Invention

The present disclosure relates to a method, a computer device and a non-transitory computer-readable memory for generating an image, and a method, a computer device and a non-transitory computer-readable medium for generating a plan, and in particular to a method, a computer device and a non-transitory computer-readable medium for generating a teeth position chart, and a method, a computer device and a non-transitory computer-readable medium for generating a treatment plan.

### 2. Description of the Related Art

Conventionally, during a diagnosis and/or treatment process for a patient, a physician frequently records conditions of a patient by means of handwriting or typing on a computer, and uses recorded results as medical records of the patient, so as to later readily explain conditions to the patient and/or evaluate conditions of the patient. Such conditions of a patient are also referred to as chief complaint (CC).

To optimize diagnosis and/or treatment time for a physician, a physician usually uses familiar terminology (for example, generally known medical terms or codes in the field) to briefly and concisely record key information related to conditions of a patient, so as to reduce the time needed for recording conditions of a patient. Later in time, the physician explains the conditions to the patient according to the recorded results, and stores the recorded results.

### BRIEF SUMMARY OF THE INVENTION

However, in practice, convenience for a physician to record conditions is primarily considered, and whether or not a patient is able to directly learn his/her conditions according to recorded results of the physician is not taken into account. Thus, the patient can only learn his/her conditions through explanation from the physician. Therefore, how to reduce cognitive differences between a patient and a physician to further enable a patient to directly read and comprehend recorded results of a physician has become issues urgently needing to be solved in the technical field.

To overcome the issues above, the present disclosure provides a method for generating a teeth position chart. The method is performed when a computer program product is loaded and executed by a computer device, so that the computer device is operable to generate a teeth position chart corresponding to medical record data after receiving the medical record data. The method includes: inputting medical record data into a first large language model; analyzing the medical record data by the first large language model, and generating and outputting an intermediate file corresponding to the medical record data; inputting the intermediate file into an image generating module; and analyzing the intermediate file by the image generating model, and generating and outputting a teeth position chart corresponding to the intermediate file. The intermediate file has at least one set of teeth position information and at least one set of symptom information respectively corresponding to the at least one set of teeth position information.

In some embodiments, the step of analyzing the medical record data by the first large language model, and generating and outputting an intermediate file corresponding to the medical record data includes: performing a first similarity comparison on the medical record data and a plurality of sets of medical record historical data stored in a historical medical record database, and generating a first similarity comparison result; retrieving at least one historical medical record from the historical medical record database according to the first similarly comparison result; performing a second similarity comparison on the medical record data and a plurality of sets of medical record result historical data stored in a historical medical record result database, and generating a second similarity comparison result; retrieving at least one historical medical record result record from the historical medical record result database according to the second similarity comparison result; and generating the intermediate file by the first large language model according to the at least one historical medical record and the at least one historical medical record result record.

In some embodiments, the step of analyzing the medical record data by the first large language model, and generating and outputting an intermediate file corresponding to the medical record data includes: performing a third similarity comparison on the medical record data and a plurality of sets of debug data in a debug database, and generating a third similarity comparison result; performing error correction on the medical record data according to the third similarity comparison result, and generating medical record correction data; and analyzing the medical record correction data by the first large language model, and generating and outputting the intermediate file corresponding to the medical record correction data.

In some embodiments, the step of analyzing the medical record correction data by the first large language model, and generating and outputting the intermediate file corresponding to the medical record correction data includes: inputting the medical record correction data to a second large language model, and determining by the second large language model whether the medical record correction data contains at least one content error; when the medical record correction data contains the at least one content error, performing error fix on the medical record correction data by the second large language model, until the medical record correction data having undergone the error fix no longer contains any content error; and when the medical record correction data does not contain any content error, analyzing the medical record correction data or the medical record correction data having undergone the error fix by the first large language model, and generating and outputting the intermediate file corresponding to the medical record correction data or the medical record correction data having undergone the error fix.

In some embodiments, the method for generating a teeth position chart further includes: comparing the intermediate data with the plurality of sets of medical record historical data in the historical medical record database, and determining whether the intermediate data contains at least one set of medical record new data different from the plurality of sets of medical record historical data; comparing the intermediate file with the plurality of sets of medical record result historical data in the historical medical record result database, and determining whether the intermediate file contains at least one set of medical record result new data different from the plurality of sets of medical record result historical data; comparing the intermediate file with the plurality of sets of debug data in the debug database, and determining whether the intermediate file contains at least one set of debug new data different from the plurality of sets of debug data; and when the intermediate file contains at least one set of medical record new data, the at least one set of medical record result new data and the at least one set of debug new data, inputting the at least one of the at least one set of medical record new data, the at least one set of medical record result new data and the at least one set of debug new data into the first large language model, and requesting the first large language model to again analyze the medical record data and to again generate and output the intermediate data, until the intermediate data again generated no longer contains any medical record new data, any medical record result new data or any debug new data.

In some embodiments, the method for generating a teeth position chart further includes: generating a treatment plan according to the teeth position chart. The treatment plan includes at least one set of teeth treatment position information and at least one treatment suggestion respectively corresponding to the at least one set of teeth treatment position information, the at least one teeth treatment position information is determined respectively according to at least one set of teeth position labeling information in the teeth position chart, and the at least one treatment suggestion is determined respectively according to at least set of symptom remark information in the teeth position chart.

In some embodiments, the method for generating a teeth position chart further includes: transmitting the treatment plan to a user terminal; receiving a rating result from the user terminal; and storing the treatment plan and the rating result to a treatment record database.

To overcome the issues above, the present disclosure further provides a computer device for generating a teeth position chart. The computer device includes a storage module and a processing module. The storage module is configured to have a computer program product stored therein. The processing module is configured to be coupled with the storage module. After the processing module loads and executes the computer program product, the processing module is operable to perform any one of the methods for generating a teeth position chart described in the present disclosure.

To overcome the issues above, the present disclosure further provides a non-transitory computer-readable recording medium for generating a teeth position chart. Once a computer device loads and executes a computer program product stored in the non-transitory computer-readable recording medium, the computer device is operable to perform any one of the methods for generating a teeth position chart described in the present disclosure.

To overcome the issues above, the present disclosure further provides a computer program product for generating a teeth position chart. Once a computer device loads and executes the computer program product, the computer device is operable to perform any one of the methods for generating a teeth position chart described in the present disclosure.

Thus, with the methods for generating a teeth position chart provided by the present disclosure, medical record data of a patient can be converted into a teeth position chart corresponding to the medical record data, so as to reduce cognitive differences between a patient and a physician, thereby enabling a patient to directly read and comprehend his/her conditions without involving explanation from a physician, as well as reducing the time needed for a physician to explain conditions to a patient. In addition, with the methods for generating a teeth position chart provided by the present disclosure, medical record data recorded by different physicians can be further converted into a teeth position chart according to a consistent standard, thereby enabling the method for generating a teeth position chart provided by the present disclosure to be suitable for multiple different physicians.

### BRIEF DESCRIPTION OF THE DRAWINGS

FIG. 1 is a block schematic diagram of a computer device for generating a teeth position chart according to an embodiment of the present disclosure.
FIG. 2 is a flowchart of a method for generating a teeth position chart according to a first embodiment of the present disclosure.
FIG. 3 is a schematic diagram of a result of a teeth position chart of the present disclosure.
FIG. 4 is a detailed flowchart of an example of step S220 shown in FIG. 2.
FIG. 5 is a detailed flowchart of another example of step S220 shown in FIG. 2.
FIG. 6 is a flowchart of a method for generating a teeth position chart according to a second embodiment of the present disclosure.
FIG. 7 is a flowchart of a method for generating a teeth position chart according to a third embodiment of the present disclosure.
FIG. 8 is a flowchart of a method for generating a treatment plan according to the first embodiment of the present disclosure.

### DETAILED DESCRIPTION OF THE INVENTION

In the following disclosure of the present disclosure, details of the present disclosure are given by way of the embodiments with reference to the accompanying drawings, so as to help a person skilled in the technical field pertinent to the present disclosure to better understand the objects, features and effects of the present disclosure.

It should be noted that, the various steps described in the present disclosure can be performed sequentially, in a reverse order, or by appropriately changing or skipping a step in the order during a control process. It should be noted that, the expression "a first step can be performed subsequent to a second step" described in the present disclosure can be interpreted that the first step follows after the second step is completely performed, or can interpreted that another step (for example, a third step) follows after the second step is completely performed and the first step follows subsequently.

Moreover, in the disclosure of the present disclosure, it should be noted that the terms such as "first", "second" and "third" are used to distinguish differences among elements, and are not to be construed as limiting to the elements themselves and specific ordering of the elements. It should be noted that, in the disclosure of the description below, the same elements or steps can be represented by the same symbols or numerals.

Moreover, the term "coupled" in the present disclosure can be interpreted as "directly connected" and/or "indirectly connected". More specifically, "a first element configured to be coupled with a second element" can be interpreted as "a first element configured to be directly connected with a second element" and/or "a first element configured to be indirectly connected with a second element".

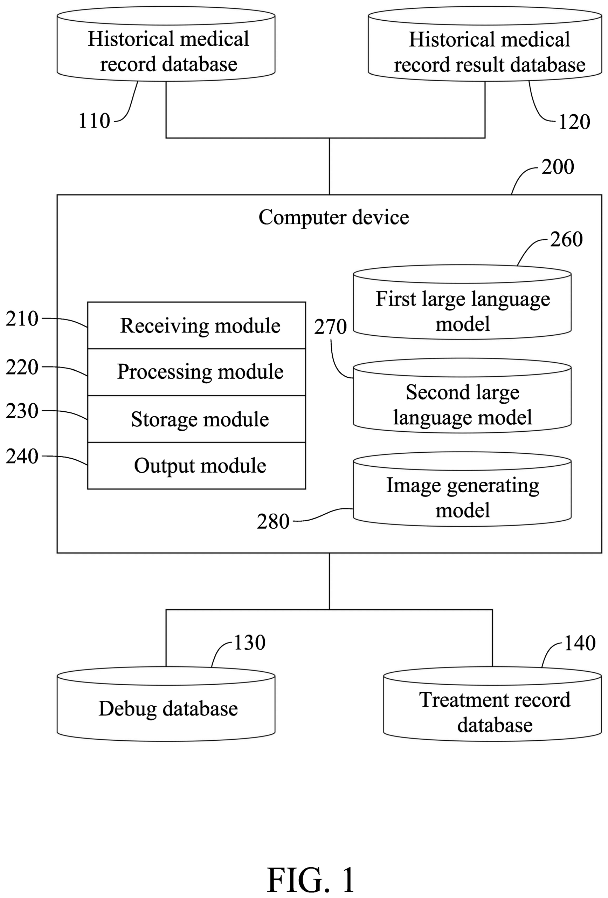

Refer to FIG. 1 showing a block schematic diagram of a computer device 200 for generating a teeth position chart according to an embodiment of the present disclosure. The computer device 200 can include a processing module 220 and a storage module 230, and can be configured to be able to convert medical record data into an intermediate file, so as to further convert the intermediate file into a teeth position chart and/or a treatment plan. In some embodiments, the computer device 200 can be, for example but not limited to, an electronic device such as a desktop computer, a notebook computer, a laptop computer, a tablet computer or other electronic devices with equivalent configurations.

The processing module 220 can be configured to be coupled with the storage module 230, and can be configured to be able to perform various steps of the method for generating a teeth position map and/or the method for generating a treatment plan described in the present disclosure. More specifically, once the processing module 220 loads and executes a computer program product, the processing module 220 is operable to perform various steps of any one of the methods for generating a teeth position map and/or the method for generating a treatment plan described in the present disclosure, thereby implementing the various steps of any one of the methods for generating a teeth position map and/or the methods for generating a treatment plan described in the present disclosure. In some embodiments, the processing module 220 can be a product generally known to a person skilled in the technical field pertinent to the present disclosure, for example but not limited to, various types of central processing units (CPU) or graphics processing units (GPU).

The storage module 230 can be configured to be coupled with the processing module 220, and can be configured to have a computer program product stored therein, so that the processing module 220 is operable to perform various steps of the methods for generating a teeth position map and/or the methods for generating a treatment plan described in the present disclosure once the processing module 220 loads and executes the computer program product. The computer program product described in the present disclosure includes a series of program codes and/or instruction sets, and more particularly, includes predetermined program codes and/or instruction sets of the methods for generating a teeth position map and/or the methods for generating a treatment plan described in the present disclosure.

In some embodiments, the storage module 230 can include one or more non-volatile memories or one or more volatile memories. In some embodiments, the volatile memory can be a product generally known to a person skilled in the technical field pertinent to the present disclosure, for example but not limited to, various types of dynamic random access memories (DRAM) or static random access memories (SRAM). In some embodiments, the non-volatile memory can be a product generally known to a person skilled in the technical field pertinent to the present disclosure, for example but not limited to, various types of read only memories (ROM) or flash memories.

In some embodiments, the computer device 200 can further include a receiving module 210 and/or an output module 240, so as to better receive medical record data of a patient from another electronic device such as a user device by the receiving module 210 and/or transmit a teeth position chart and/or a treatment plan to another electronic device such as a user device by the output module 240. In some embodiments, the receiving module 210 can be configured to be coupled with the processing module 220, and the output module 240 can be configured to be coupled with the processing module 220.

In some embodiments, the computer device 200 can be configured to be coupled with another electronic device (not shown) such as a user device and/or a server by a physical signal line and/or a virtual signal line, so that the computer device 200 can receive medical record data of a patient from another electronic device through the physical signal line and/or the virtual signal line, and can transmit a teeth position chart and/or a treatment plan generated by the processing module 220 to another electronic device through the physical signal line and/or the virtual signal line. In some embodiments, the physical signal line can be, for example but not limited to, a network signal line compliant with the Internet protocol. In some embodiments, the virtual signal line can be, for example but not limited to, Wi-Fi, 4G/5G/6G/, Bluetooth, Near-Field Communication (NFC) compliant with wireless communication protocols.

In some embodiments, the first large language model 260 can be further integrated into the computer device 200. The computer device 200 can input medical record data of a patient to the first large language model 260, and then text contents in the medical record data are analyzed by the first large language model 260 to generate and output the intermediate file corresponding to the medical record data. The intermediate file generated and output has at least one set of teeth position information and at least one set of symptom information respectively corresponding to the at least one set of teeth position information. In some embodiments, the at least one set of symptom information can include, for example but not limited to, missing, bridge, impaction or 3rd molar, pulpitis, residual root, caries, extraction and/or root-endo.

In some embodiments, the medical record data of a patient can be, for example but not limited to, chief complaint (CC). More specifically, the medical record data of a patient can be, for example but not limited to, "CC: Asking for dental check-up. Visual and radiographic examination. Dx&Tx plan: Panoramic Finding (34004) 11 angular bony defect , 12 veneer , 13 missing , 14 extraction , 15 crown , 16 implant 26 inlay , 25 onlay , 24 overlay , 23 normal , 21 apical surgery without endo , 22 periapical lesion 36 removal , 35 pending , 31 perio-endo combined lesion , 33 apical surgery , 34 impaction , 32 separated instrument , 37 DL caries 41 endo , 42 post , 43 vital pulp therapy , 47 fracture , 44 X 46 bridge".

In some embodiments, the first large language model 260 can be, for example but not limited to, ChatGPT, Bard, Claude or LLaMa. Since the first large language model 260 is a large deep learning model and has the ability to analyze semantics of a string based on a small number of string hints to provide a prediction, the first large language model 260 is capable of generating and outputting the intermediate file corresponding the medical record data according to the medical record data of a patient. In some embodiments, the intermediate file can be, for example, a file in a JavaScript Object Notation (JSON) format, so that the medical record data can be converted into a file that can be more easily read and processed. In some other embodiments, the intermediate file can also be a file compliant with other formats, for example, in a data structure in Extensible Markup Language (XML), Comma-Separated Values (CVS), XLSX, Structured Query Language (SQL), Health Level 7 (HL7), an array or a list.

In some embodiments, the image generating model 280 can be further integrated into the computer device 200. The computer device 200 can input the intermediate file into the image generating model 280, and then text contents in the intermediate file are analyzed by the image generating model 280 to generate and output a teeth position chart corresponding to the intermediate file. In some embodiments, the image generating model 280 can be, for example but not limited to, a function for generating a teeth position chart. Taking a function for generating a teeth position chart for example, once the intermediate file is input into the image generating model 280, the image generating model 280 can perform a corresponding rendering operation on an initial blank teeth position chart according to the text contents in the intermediate file, so as to generate the teeth position chart corresponding to the intermediate file. More specifically, the image generating model 280 can traverse the numbering of all the teeth in the intermediate file to make corresponding modifications and/or drawings on a blank chart of the initial state of the teeth position according to the corresponding state of each tooth in the intermediate file, so that the image generating model 280 can generate and output the teeth position chart corresponding to the intermediate file according to the contents of the intermediate file. For example, if the state of the 31st tooth is Missing, the image generating model 280 can make corresponding modifications and/or drawings on a blank chart of the initial state of the teeth position, i.e., erase the existing tooth pattern corresponding to the 31st tooth, in order to generate a teeth position chart with the 31st tooth as a missing tooth. Further, if the state of the 13th, 14th, 15th and 16th teeth is a bridge, the image generating model 280 can make corresponding modifications and/or drawings on a blank chart of the initial state of the teeth position, i.e., adding a pattern of a bridge marking at the tooth pattern corresponding to the 13th, 14th, 15th and 16th teeth, in order to generate a teeth position chart with the 13th, 14th, 15th and 16th teeth as a bridge state. The teeth position chart generated and output has at least one set of teeth position labeling information and at least one set of symptom remark information respectively corresponding to the at least one set of teeth position labeling information.

In some embodiments, the second large language model 270 can be further integrated into the computer device 200. The computer device 200 can input medical record correction data into the second large language model 260, and then text contents in the medical record correction data are analyzed by the second large language model 260 to determine whether the medical record correction data contains at least one content error. In some embodiments, the second large language model 270 can be, for example but not limited to, ChatGPT, Bard, Claude or LLaMa. Since the second large language model 270 is a large deep learning model and has the ability to analyze semantics of a string based on a small number of string hints to provide a prediction, the second large language model 270 is capable of determining whether the medical record correction data contains at least one content error. When the medical record correction data contains the at least one content error, the second large language model 270 can further predict a possible correct answer, so as to perform error fix for the at least one content error in the medical record correction data, and to further generate and output medical record correction data having undergone the error fix.

In some embodiments, the computer device 200 can be further configured to couple with various databases such as the historical medical record database 110, the historical medical record result database 120, the debug database 130 and a treatment record database 140, so as to readily read various types of data from the various databases above and/or store various types of data to the various of databases above. For example, the computer device 200 can read a plurality of sets of medical record historical data from the historical medical record database 110, read a plurality of sets of medical record result historical data from the historical medical record result database 120, and read a plurality of sets of debug data from the debug database 130. Moreover, the computer device 200 can store the medical record data, the intermediate file, the teeth position chart and/or the treatment plan to a database such as the treatment record database 140.

With the computer device 200 shown in FIG. 1, any one of the methods for generating a teeth position chart and/or the methods for generating a treatment plan described in the present disclosure can be implemented, and medical record data can be converted into an intermediate file to further convert the intermediate file into a teeth position chart and/or a treatment plan, thereby enabling a patient to directly read and comprehend his/her conditions and/or the treatments regarding the conditions without involving explanation from a physician, as well as reducing the time needed for a physician to explain conditions to a patient.

In some embodiments, a person of ordinary skill in the technical field pertinent to the present disclosure can implement any one of the methods for generating a teeth position chart and/or the methods for generating a treatment plan described in the present disclosure under a development environment such as Windows 10/11, MacOS and Ubuntu with the coordination of toolkits such as Visual Studio Code by. Moreover, a person of ordinary skill in the technical field pertinent to the present disclosure can use a program language such as Python and Javascript with reference to a function database such as OpenAI, Firebase, Google Cloud, tiktoken and React.js to complete a computer program product, so as to implement any one of the methods for generating a teeth position chart and/or the methods for generating a treatment plan described in the present disclosure. In some embodiments, a person of ordinary skill in the technical field pertinent to the present disclosure can use such as Firebase and Pinecone to build various types of databases such as the historical medical record database 110, the historical medical record result database 120, the debug database 130 and the treatment record database 140.

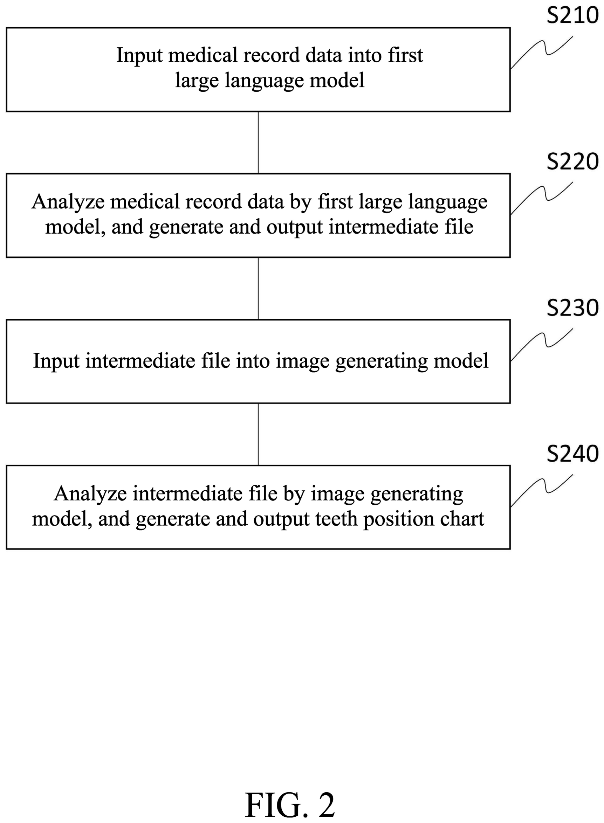

FIG. 2 shows a flowchart of a method for generating a teeth position chart according to a first embodiment of the present disclosure. The method shown in FIG. 2 can be performed by the processing module 220 of the computer device 200 shown in FIG. 1, so as to convert medical record data into an intermediate file to further convert the intermediate file into a teeth position chart. The method shown in FIG. 2 can include steps S210, S220, S230 and S240.

In some embodiments, taking FIG. 2 for example, step S220 can be performed after step S210, step S230 can be performed after step S220, and step S240 can be performed after step S230.

In step S210, once the computer device 200 receives the medical record data of a patient, the computer device 200 can input the medical record data received into the first large language model 260, so as to analyze the text contents in the received medical record data by the first large language model 260. In some embodiments, the computer device 200 can receive the medical record data of a patient by various means, for example but not limited to, from text input by a user, a speech input by a user and/or a text file input by a user. In some embodiments, the medical record data of a patient can be, for example but not limited to, chief complaint (CC). In some embodiments, the first large language model 260 can be, for example but not limited to, ChatGPT, Bard, Claude or LLaMa.

In step S220, once the medical record data is input into the first large language model 260, the computer device 200 can analyze the text contents in the input medical record data input by the first large language model 260, so as to generate and output the intermediate file corresponding to the input medical record data. In some embodiments, the intermediate file can be, for example, a file in a JavaScript Object Notation (JSON) format, so that the medical record data can be converted into a file that can be more easily read and processed. The intermediate file generated and output has at least one set of teeth position information and at least one set of symptom information respectively corresponding to the at least one set of teeth position information.

In step S230, the computer device 20 can input the intermediate file corresponding into the medical record data to the image generating model 280, so as to analyze the text contents in the intermediate file by the image generating model 280. In some embodiments, the image generating model 280 can be, for example but not limited to, a function for generating a teeth position chart. The image generating model 280 can perform a corresponding rendering operation on an initial blank teeth position chart according to the text contents in the intermediate file, so as to generate a teeth position chart corresponding to the intermediate file. More specifically, the image generating model 280 can traverse the numbering of all the teeth in the intermediate file to make corresponding modifications and/or drawings on a blank chart of the initial state of the teeth position according to the corresponding state of each tooth in the intermediate file, so that the image generating model 280 can generate and output the teeth position chart corresponding to the intermediate file according to the contents of the intermediate file. For example, if the state of the 31st tooth is Missing, the image generating model 280 can make corresponding modifications and/or drawings on a blank chart of the initial state of the teeth position, i.e., erase the existing tooth pattern corresponding to the 31st tooth, in order to generate a teeth position chart with the 31st tooth as a missing tooth. Further, if the state of the 13th, 14th, 15th and 16th teeth is a bridge, the image generating model 280 can make corresponding modifications and/or drawings on a blank chart of the initial state of the teeth position, i.e., adding a pattern of a bridge marking at the tooth pattern corresponding to the 13th, 14th, 15th and 16th teeth, in order to generate a teeth position chart with the 13th, 14th, 15th and 16th teeth as a bridge state. The teeth position chart generated and output has at least one set of teeth position labeling information and at least one set of symptom remark information respectively corresponding to the at least one set of teeth position labeling information. The at least one set of teeth position labeling information in the teeth position chart corresponds to at least one set of teeth position information in the intermediate file, and the at least one set of symptom remark information in the teeth position chart corresponds to at least one set of symptom information in the intermediate file.

In step S240, once the intermediate file corresponding to the medical record data is input into the image generating model 280, the computer device 200 can analyze text contents in the intermediate file by the image generating model 280, so as to generate and output a teeth position chart corresponding to the input intermediate file.

With the method shown in FIG. 2, medical record data can be converted into an intermediate file to further convert the intermediate file into a teeth position chart, thereby enabling a patient to directly read and comprehend his/her conditions without involving explanation from a physician, as well as reducing the time needed for a physician to explain conditions to a patient. In addition, with the method shown in FIG. 2, medical record data recorded by different physicians can be further converted into a teeth position chart according to a consistent standard, thereby enabling the method for generating a teeth position chart provided by the present disclosure to be suitable for multiple different physicians.

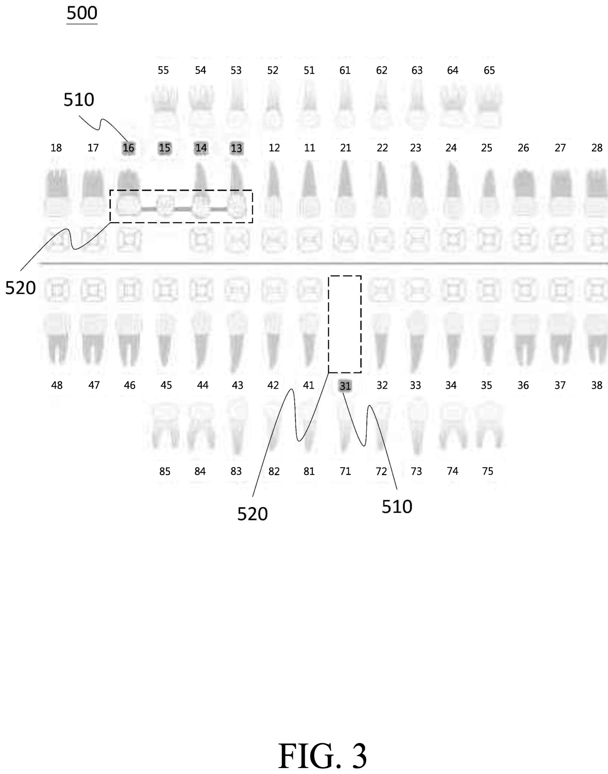

FIG. 3 shows a schematic diagram of a result of a teeth position chart 500 of the present disclosure. That is to say, after the computer device 200 completely performs the method for generating a teeth position chart shown in FIG. 2, the computer device 200 can generate and output the teeth position chart 500 shown in FIG. 3.

More specifically, once the computer device 200 receives the above medical record data of a patient as shown in Table-1 below, the computer device 200 can generate and output the above intermediate file corresponding to the medical record data, as shown in Table-2 below. Taking Table-2 below for example, the intermediate file can include at least one set of teeth position information (that is, 13, 14, 15, 16 and 31) and at least one set of symptom information (that is, missing and bridge) corresponding to the at least one set of teeth position information.

**Table-1: Medical record data of patient**

| |
|---|
| Dx&Tx plan: Panoramic Finding (00315C) |
| - Missing: 31 |
| - Impaction: |
| - Impaction: |
| - Crown/Bridge/SSC: 16-x-14-13 |
| - Pulpitis: |
| - RR: |
| - Caries: |
| - Periodontal status: [ ] Normal, [ ] Gingivitis, [ ] Periodontitis |
| - Other finding: |

**Table-2: Intermediate file corresponding to medical record data**

| |
|---|
| { |
| "31": "Missing", |
| "16, 15, 14, 13": "Bridge", |
| "15": "Missing" |
| } |

Next, the computer device 200 can generate and output the teeth position chart 500 shown in FIG. 3 by the image generating model 280. Taking FIG. 3 for example, the teeth position chart 500 can include at least one set of teeth position labeling information 510 and at least one set of symptom remark information 520, wherein the at least one set of teeth position labeling information 510 respectively corresponds to the at least one teeth position information in the intermediate file, and the at least one set of symptom remark information 520 respectively corresponds to the at least one set of symptom information in the intermediate file. In addition, the computer device 200 can present, in form of an image on the teeth position chart 500, the at least one set of symptom information (that is, the at least one set of symptom remark information 520) in the intermediate file. As such, a patient is able to clearly learn at a glance teeth conditions of the patient directly according to the teeth position chart 500 without involving explanation from a physician, thereby reducing cognitive differences between a patient and a physician, as well as reducing the time needed for a physician to explain conditions to a patient.

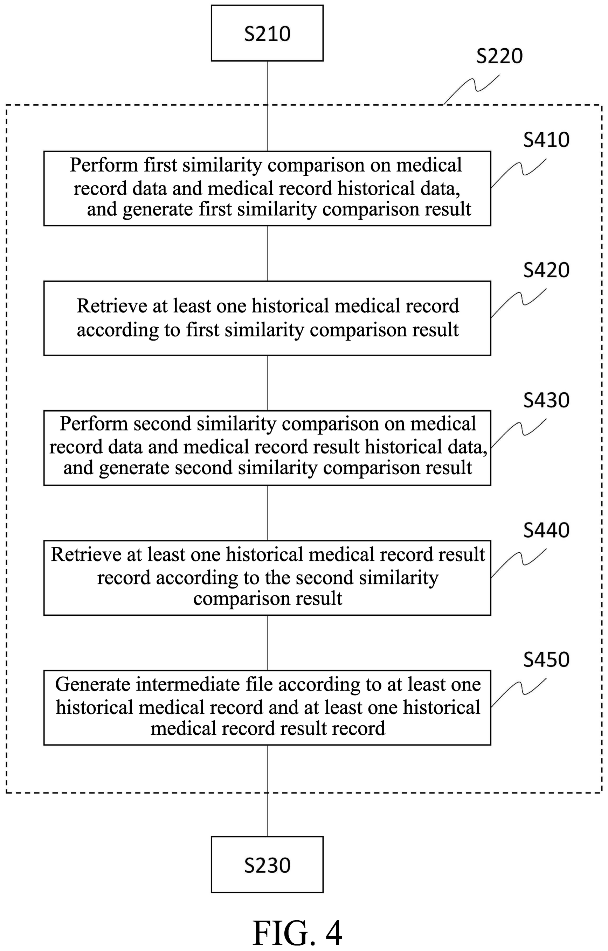

Refer to FIG. 4 showing a detailed flowchart of an example of step S220 shown in FIG. 2. That is to say, step S220 shown in FIG. 2 can be completed further by performing steps S410, S420, S430, S440 and S450.

In some embodiments, taking FIG. 4 for example, step S410 can be performed after step S210, step S420 can be performed after step S410 and step S430 can be performed after step S420, step S440 can be performed after step S430, step S450 can be performed after step S440, and step S230 can be performed after step S450. In some embodiments, step S410 and step S430 can be performed simultaneously at a same timing.

In step S410, the computer device 200 can read a plurality of sets of medical record historical data from the historical medical record database 110, and then perform a first similarity comparison on the received medical record data and each set of medical record historical data of the plurality of sets of medical record historical data, so as to further generate a corresponding first similarity comparison result for each set of medical record historical data of the plurality of sets of medical record historical data. In some embodiments, a person of ordinary skill in the technical field pertinent to the present disclosure can use a vector data comparison method of a Word2vec model related to the word embedding technology to individually analyze a text similarity between the medical record data and each set of medical record historical data. In some other embodiments, a person of ordinary skill in the technical field pertinent to the present disclosure can also use methods such as, for example but not limit to, cosine similarity, term frequency-inverse document frequency (TF-IDF), Jaccard similarity, Levenshtein distance, an optimally matched Okapi BM25 model, bidirectional encoder representations from transformers (BERT), or byte pair encoding (BPE), to perform the first similarity comparison. In some embodiments, the first similarity comparison result can be presented in form of percentage.

In step S420, the computer device 200 can retrieve at least one historical medical record from the historical medical record database 110 according to the first similarity comparison result. More specifically, the computer device 200 can retrieve at least one historical medical record of which the first similarity comparison result is greater than or equal to 30%, more preferably, of which the first similarity comparison result is greater than or equal to 40%, and most preferably of which the first similarity comparison result is greater than or equal to 50%, from the historical medical record database 110. As such, the computer device 200 can retrieve a historical medical record highly similar to the medical record data from the historical medical record database 110 according to the first similarity comparison result.

In step S430, the computer device 200 can read a plurality of sets of medical record result historical data from the historical medical record result database 120, and then perform a second similarity comparison on the received medical record data and each set of medical record result historical data of the plurality of sets of medical record result historical data, so as to further generate a corresponding second similarity comparison result for each set of medical record result historical data of the plurality of sets of medical record result historical data. In some embodiments, a person of ordinary skill in the technical field pertinent to the present disclosure can use a vector data comparison method of a Word2vec model related to the word embedding technology to individually analyze a text similarity between the medical record data and each set of medical record result historical data. In some other embodiments, a person of ordinary skill in the technical field pertinent to the present disclosure can also use methods such as, for example but not limit to, cosine similarity, term frequency-inverse document frequency (TF-IDF), Jaccard similarity, Levenshtein distance, an optimally matched Okapi BM25 model, bidirectional encoder representations from transformers (BERT), or byte pair encoding (BPE), to perform the second similarity comparison. In some embodiments, the second similarity comparison result can be presented in form of percentage.

In step S440, the computer device 200 can retrieve at least one historical medical record result record from the historical medical record result database 120 according to the second similarity comparison result. More specifically, the computer device 200 can retrieve at least one historical medical record result record of which the second similarity comparison result is greater than or equal to 50%, more preferably, of which the second similarity comparison result is greater than or equal to 70%, and most preferably of which the second similarity comparison result is greater than or equal to 80%, from the historical medical record result database 120. As such, the computer device 200 can retrieve a historical medical record result record highly similar to the medical record data from the historical medical record result database 120 according to the second similarity comparison result.

In step S450, the computer device 200 can generate the intermediate file according to the at least one historical medical record and the at least one historical medical record result record. More specifically, the computer device 200 can generate and output the intermediate file by the first large language model 260 according to the at least one historical medical record and the at least one historical medical record result record.

With the method shown in FIG. 4, the existing plurality of sets of medical record historical data and plurality of medical record result historical data can be properly utilized, so as to enable the computer device 200 to be able to generate and output the intermediate file with a higher prediction rate according to the plurality of sets of medical record historical data and the plurality of set of medical record result historical data, and to enhance the accuracy and referenceability of a teeth position chart and/or treatment plan subsequently generated.

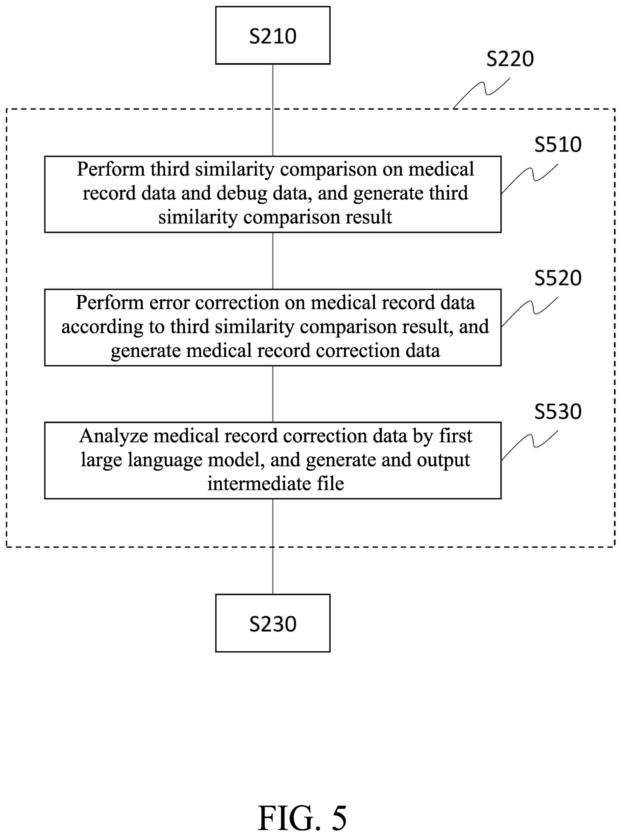

Refer to FIG. 5 showing a detailed flowchart of another example of step S220 shown in FIG. 2. That is to say, step S220 shown in FIG. 2 can be completed further by further performing steps S510, S520 and S530.

In some embodiments, taking FIG. 5 for example, step S510 can be performed after step S210, step S520 can be performed after step S510, step S530 can be performed after step S520, and step S230 can be performed after step S530.

In step S510, the computer device 200 can read a plurality of sets of debug data from the debug database 130, and then perform a third similarity comparison on the received medical record data and each set of debug data of the plurality of sets of debug data, so as to further generate a corresponding third similarity comparison result for each set of debug data of the plurality of sets of debug data. In some embodiments, a person of ordinary skill in the technical field pertinent to the present disclosure can use a vector data comparison method of a Word2vec model related to the word embedding technology to individually analyze a text similarity between a keyword of the medical record data and each set of debug data. In some other embodiments, a person of ordinary skill in the technical field pertinent to the present disclosure can also use methods such as, for example but not limit to, cosine similarity, term frequency-inverse document frequency (TF-IDF), Jaccard similarity, Levenshtein distance, an optimally matched Okapi BM25 model, bidirectional encoder representations from transformers (BERT), or byte pair encoding (BPE), to perform the third similarity comparison. In some embodiments, the third similarity comparison result can be presented in form of percentage.

In step S520, the computer device 200 can perform error correction on the medical record data according to the third similarity comparison result, so as to generate medical record correction data. More specifically, when a keyword of the computer device 200 in the medical record data is an error, the computer device 200 can use debug data of which the third similarity result is the highest (or closest to 100%) to perform the error correction on the medical record data. For example, a term such as "gumbs" can be error fixed to "gums" by using the debug data or which the third similarity comparison result is the highest, and the term "embedded toothe" in the medical record data can also be error fixed to "embedded tooth" by using the debug data of which the third similarity comparison result is the highest. As such, the computer device 200 can perform error correction on the medical record data by performing step S510 and step S520, so as to generate medical record correction data.

In step S530, the computer device 200 can analyze text contents in the medical record correction data by the first large language model 260, so as to generate and output the intermediate file corresponding to the medical record correction data. In some embodiments, the intermediate file can be, for example, a file in a JavaScript Object Notation (JSON) format, so that the medical record data can be converted into a file that can be more easily read and processed.

In some embodiments, the computer device 200 can input the medical record correction data into the second large language model 260, and then the text contents in the medical record correction data are analyzed by the second large language model 260 to determine whether the medical record correction data contains at least one content error. When the second large language model 270 determines that the medical record correction data contains the at least one content error, the computer device 200 can perform error fix on the medical record correction data by the second large language model. When the second large language model 270 determines that the medical record correction data having undergone the error fix still contains the at least one content error, the computer device 200 can again perform the error fix on the medical record correction data having undergone the error fix by the second large language model 270, until the medical record correction data having undergone the error fix no longer contains any content error. When the second large language model 270 determines that the medical record correction data or the medical record correction data having undergone the error fix does not contain any content error, the computer device 200 can analyze the text contents in the medical record correction data or the text contents in the medical record correction data having undergone the error fix by the first large language model 260, so as to generate and output the intermediate file corresponding to the medical record correction data or the medical record correction data having undergone the error fix. In some embodiments, the second large language model 270 can be, for example but not limited to, ChatGPT, Bard, Claude or LLaMa.

With the method shown in FIG. 5, incorrect analysis or output result caused by an error in the medical record data can be effectively prevented, so as enable the computer device 200 to be able to generate and output the intermediate file with a higher prediction rate according to the medical record correction data or the medical record correction data having undergone the error fix, and to enhance the accuracy and referenceability of a teeth position chart and/or treatment plan subsequently generated.

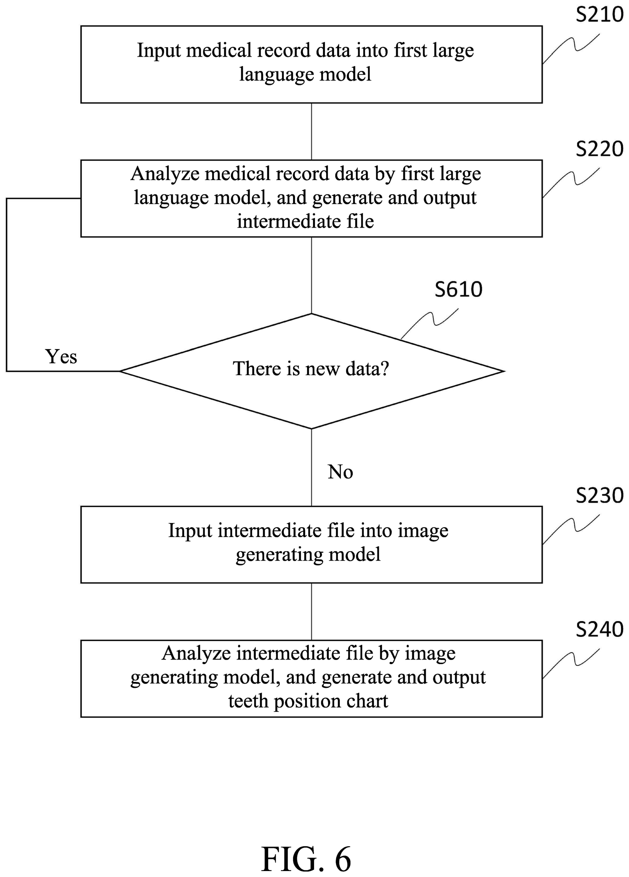

Refer to FIG. 6 showing a flowchart of a method for generating a teeth position chart according to a second embodiment of the present disclosure. The method shown in FIG. 6 can include steps S210, S220, S230, S240 and S610, wherein steps S210, S220, S230 and S240 can be substantially the same as those shown in FIG. 2. In some embodiments, taking FIG. 6 for example, step S610 can be performed after step S220.

In step S610, the computer device 200 can determine whether the intermediate file contains new data. More specifically, the computer device 200 can read a plurality of set of medical record historical data from the historical medical record database 110, and then compare the text contents in the intermediate file with each set of medical record historical data of the plurality of sets of medical record historical data, to further determine whether the intermediate file contains at least one set of medical record new data different from the plurality of sets of medical record historical data. The computer device 200 can read a plurality of sets of medical record result historical data from the historical medical record result database 120, and then compare the text contents in the intermediate file with each set of medical record result historical data of the plurality of sets of medical record result historical data, to determine whether the intermediate file contains at least one set of medical record new data different from the plurality of sets of medical record result historical data. The computer device 200 can read a plurality of sets of debug data from the debug database 130, and then compare the text contents in the intermediate file with each set of debug data of the plurality of sets of debug data, to determine whether the intermediate file contains at least one set of debug new data different from the plurality of sets of debug data.

When the computer device 200 determines that the intermediate file contains at least one of the at least one set of medical record new data, the at least one set of medical record result new data and the at least one set of debug new data, the computer device 200 can further request the first large language model 260 to again analyze the medical record data, so as to again generate and output a new intermediate file. When the computer device 200 determines that the new intermediate file still contains at least of at least one of the at least one set of medical record new data, the at least one set of medical record result new data and the at least one set of debug new data, the computer device 200 can further request the first large language model 260 to again analyze the medical record data, so as to again generate and output a new intermediate file, until the new intermediate file no longer contains any medical record new data, any medical record result new data or any debug new data. When the computer device 200 determines that the intermediate file does not contain any medical record new data, any medical record result new data or any debug new data, the computer device 200 can subsequently perform step S230 and step S240, so as to generate and output a teeth position chart corresponding to the intermediate file or the new intermediate file.

The method shown in FIG. 6 can effectively ensure that the generated intermediate file does not exceed user-expected medical record new data, medical record result new data or debug new data, so that the computer device 200 is capable of generating and outputting a subsequent teeth position chart and/or a treatment plan according to the intermediate file meeting user expectations, and enhancing stability of the teeth position chart and/or treatment plan subsequently generated.

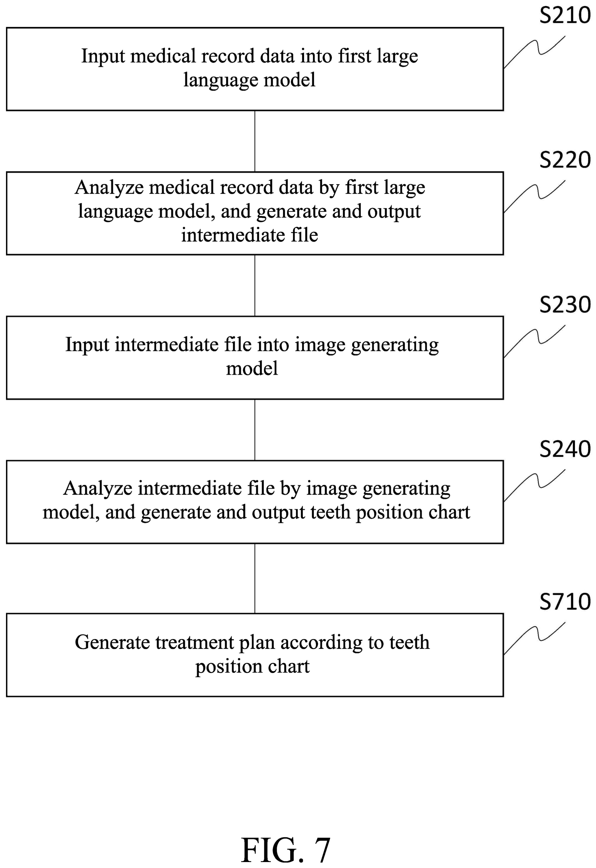

Refer to FIG. 7 showing a flowchart of a method for generating a teeth position chart according to a third embodiment of the present disclosure. The method shown in FIG. 7 can include steps S210, S220, S230, S240 and S710, wherein steps S210, S220, S230 and S240 can be substantially the same as those shown in FIG. 2. In some embodiments, taking FIG. 7 for example, step S710 can be performed after step S240.

In step S710, the computer device 200 can generate a corresponding treatment plan according to the teeth position plan, wherein the treatment plan has at least one set of teeth treatment position information and at least one treatment suggestion respectively corresponding to the at least one set of teeth treatment position information. In some embodiments, the at least one set of teeth treatment position information in the treatment plan is determined respectively according to the at least one set of teeth position labeling information in the teeth position chart, and the at least one treatment suggestion is determined respectively according to the at least set of symptom remark information in the teeth position chart.

For example, when the at least one set of symptom remark information in the teeth position chart indicates "missing", the at least one treatment suggestion in the treatment plan can be correspondingly determined as "implant", "crown&bridge (C&B)", "guide bone regeneration (GBR)", or "guided tissue regeneration (GTR)". When the at least one set of symptom remark information in the teeth position chart indicates "impaction or 3rdMolor", the at least one treatment suggestion in the treatment plan can be correspondingly determined as "extraction". When the at least one set of symptom remark information in the teeth position chart indicates "pulpitis", the at least one treatment suggestion in the treatment plan can be correspondingly determined as "endodontics". When the at least one set of symptom remark information in the teeth position chart indicates "residual root", the at least one treatment suggestion in the treatment plan can be correspondingly determined as "extraction". When the at least one set of symptom remark information indicates "caries", the at least one treatment suggestion in the treatment plan can be correspondingly determined as "operative dentistry (OD)", "vital pulp therapy (VPT)", "endodontics", or "extraction". When the at least one set of symptom remark information in the teeth position chart indicates "extraction", the at least one treatment suggestion in the treatment plan can be correspondingly determined as "guided bone regeneration (GBR)", or "guided tissue regeneration (GTR)". When the at least one set of symptom remark information in the teeth position chart indicates "root-endo", the at least one treatment suggestion in the treatment plan can be correspondingly determined as "post", "crown", "bonded porcelain restoration (BPR)", or "operative dentistry (OD)". When the at least one set of symptom remark information in the teeth position chart indicates "bridge", the at least one treatment suggestion in the treatment plan can be correspondingly determined as "crown & bridge (C&B)".

With the method in FIG. 7, in addition to generating a teeth position chart having teeth position labeling information and symptoms remark information, a treatment plan having teeth treatment position information and treatment suggestions can be further generated, so that a patient is able to clearly learn teeth conditions and/or treatment details needing to be performed for teeth conditions of the patient without involving explanation from a physician, thereby reducing cognitive differences between a patient and a physician, as well as reducing the time needed for a physician to explain conditions to a patient.

Taking the teeth position chart 500 in FIG. 3 for example, after the computer device 200 performs step S710, the computer device 200 can generate the above treatment plan corresponding to the teeth position chart 500 according to the teeth position chart 500.

**Table-3: Treatment plan corresponding to teeth position chart 500**

| Teeth treatment Position information | Treatment suggestion | Teeth treatment Position information | Treatment suggestion |
|---|---|---|---|
| 13 | Crown & Bridge (C&B) | 15 | Guided tissue regeneration (GTR) |
| 14 | Crown & Bridge (C&B) | 15 | Sinus lifting |
| 15 | Crown & Bridge (C&B) | 31 | Implant |
| 16 | Crown & Bridge (C&B) | 31 | Crown & Bridge (C&B) |
| 15 | Implant | 31 | Guided bone regeneration (GBR) |
| 15 | Guided bone regeneration (GBR) | 31 | Guided tissue regeneration (GTR) |

In some embodiments, the at least one set of teeth position labeling information in the teeth position chart respectively corresponds to the at least one set of teeth position information in the intermediate file, and the at least one set of symptom remark information in the teeth position chart respectively corresponds to at least one set of symptom information in the intermediate file. Thus, the computer device 200 can also directly generate a treatment plan corresponding to the intermediate file according to the intermediate file.

In some other embodiments, the computer device 200 can further transmit the treatment plan to a user terminal. In some embodiments, the computer device 200 can be, for example but not limited to, an electronic device such as a desktop computer, a notebook computer, a laptop computer, a tablet computer, a smart watch, a wearable device or other electronic devices with equivalent configurations. Next, after a user rates the treatment plan received by the user terminal, the computer device 200 can further receive a rating result corresponding to the treatment plan from the user terminal. Next, the computer device 200 can further store the treatment plan and the rating result together to the treatment record database 140. Thus, with the various steps above, related data can be stored in databases in a more diversified manner, thereby enhancing operation flexibilities of subsequent utilization of the related data.

In addition, the computer device 200 can use the treatment plan and the rating result as training data for the first large language model 260, so as to adjust a calculation weighting of the first large language model 260 according to the treatment plan and the rating result, thereby improving prediction accuracy of the first large language model 260 to better generate more accurate intermediate file and subsequent teeth position chart and/or treatment plan.

In some embodiments, the various step in the method for generating a teeth position plan described in the present disclosure can be further combined, replaced, repeated and/or modified, so as to form new embodiments without going beyond the scope disclosed by the present disclosure.

In some embodiments, the various steps of the method for generating a teeth position chart described in the present disclosure can be stored in a non-transitory computer-readable recording medium, which can be, for example but not limited to, a hard drive, an optic disk, a magnetic disk, a portable drive or a network-accessible database. When a computer program product stored in the non-transitory computer-readable recording medium is loaded and execute by a computer device, the computer device is operable to perform any one of the methods for generating a teeth position chart described in the present disclosure.

In some embodiments, a computer program product for generating a teeth position chart can include the various steps of the methods for generating a teeth position chart described in the present disclosure. When a computer device loads and executes the computer program product, any one method of the methods for generating a teeth position chart described in the present disclosure can be implemented.

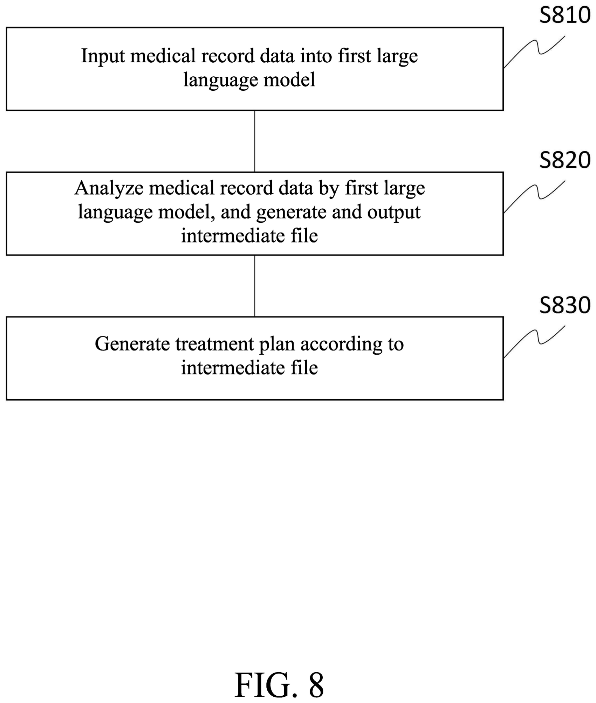

Refer to FIG. 8 showing a flowchart of a method for generating a treatment plan according to the first embodiment of the present disclosure. The method shown in FIG. 8 can be performed by the processing module 220 of the computer device 200 shown in FIG. 1, so as to convert medical record data into an intermediate file to further convert the intermediate file into a treatment plan. The method shown in FIG. 8 can include steps S810, S820 and S830.

In step S810, once the computer device 200 receives the medical record data of a patient, the computer device 200 can input the medical record data received into the first large language model 260, so as to analyze the text contents in the received medical record data by the first large language model 260. In some embodiments, the computer device 200 can receive the medical record data of a patient by various means, for example but not limited to, from text input by a user, a speech input by a user and/or a text file input by a user. In some embodiments, the medical record data of a patient can be, for example but not limited to, chief complaint (CC). In some embodiments, the first large language model 260 can be, for example but not limited to, ChatGPT, Bard, Claude or LLaMa.

In step S820, once the medical record data is input into the first large language model 260, the computer device 200 can analyze the text contents in the input medical record data input by the first large language model 260, so as to generate and output the intermediate file corresponding to the input medical record data. In some embodiments, the intermediate file can be, for example, a file in a JavaScript Object Notation (JSON) format, so that the medical record data can be converted into a file that can be more easily read and processed. The intermediate file generated and output has at least one set of teeth position information and at least one set of symptom information respectively corresponding to the at least one set of teeth position information.

In step S830, the computer device 200 can generate the corresponding treatment plan according to the intermediate file, wherein the treatment plan has at least one set of teeth treatment position information and at least one treatment suggestion respectively corresponding to the at least one set of teeth treatment position information. In some embodiments, the at least one set of teeth treatment position information in the treatment plan is determined respectively according to the at least one set of teeth position information in the intermediate file, and the at least one treatment suggestion is determined respectively according to the at least set of symptom information in the intermediate file.

For example, when the at least one set of symptom information in the intermediate file indicates "missing", the at least one treatment suggestion in the treatment plan can be correspondingly determined as "implant", "crown&bridge (C&B)", "guide bone regeneration (GBR)", or "guided tissue regeneration (GTR)". When the at least one set of symptom information in the intermediate file indicates "impaction or 3rdmolar", the at least one treatment suggestion in the treatment plan can be correspondingly determined as "extraction". When the at least one set of symptom information in the intermediate file indicates "pulpitis", the at least one treatment suggestion in the treatment plan can be correspondingly determined as "endodontics". When the at least one set of symptom information in the intermediate file indicates "residual root", the at least one treatment suggestion in the treatment plan can be correspondingly determined as "extraction". When the at least one set of symptom information in the intermediate file indicates "caries", the at least one treatment suggestion in the treatment plan can be correspondingly determined as "operative dentistry (OD"), vital pulp therapy (VPT)", "endodontics", or "extraction". When the at least one set of symptom information in the intermediate file indicates "extraction", the at least one treatment suggestion in the treatment plan can be correspondingly determined as "guided bone regeneration (GBR)", or "guided tissue regeneration (GTR)". When the at least one set of symptom information in the intermediate file "root-endo", the at least one treatment suggestion in the treatment plan can be correspondingly determined as "post", "crown", "bonded porcelain restoration (BPR)", or "operative dentistry (OD)". When the at least one set of symptom information in the intermediate file "bridge", the at least one treatment suggestion in the treatment plan can be correspondingly determined as "crown & bridge (C&B)".

With method shown in FIG. 8, medical record data can be converted into an intermediate file to further convert the intermediate file into a treatment plan, thereby enabling a patient to directly learn treatment details needing to be performed for his/her conditions without involving explanation from a physician, as well as reducing the time needed for a physician to explain conditions to a patient. In addition, with the method shown in FIG. 8, medical record data recorded by different physicians can be further converted into a treatment plan according to a consistent standard, thereby enabling the method for generating a treatment plan provided by the present disclosure to be suitable for multiple different physicians.

In some embodiments, the various steps of the method for generating a treatment plan described in the present disclosure can be stored in a non-transitory computer-readable recording medium, which can be, for example but not limited to, a hard drive, an optic disk, a magnetic disk, a portable drive or a network-accessible database. When a computer program product stored in the non-transitory computer-readable recording medium is loaded and execute by a computer device, the computer device is operable to perform any one of the methods for generating a treatment plan described in the present disclosure.

In some embodiments, a computer program product for generating a treatment plan can include the various steps of the methods for generating a treatment plan described in the present disclosure. Once a computer device loads and executes the computer program product, any one method of the methods for generating a treatment plan described in the present disclosure can be implemented.

Although the present invention is described further in detail by way of specific embodiments and the accompanying drawings, numerous modifications and changes may be made by a person skilled in the technical field pertinent to the present invention without departing from the scope or spirit defined in the appended claims. Therefore, the scope of protection of the present invention should be accorded with the broadest interpretation of the appended claims and shall not be restricted by the disclosure of the detailed description.

## Claims

1. A method for generating a teeth position chart, the method performed when a computer program product is loaded and executed by a computer device, so that the computer device is operable to generate the teeth position chart corresponding to medical record data after receiving the medical record data, the method comprising:
inputting the medical record data into a first large language model;
analyzing the medical record data by the first large language model, and generating and outputting an intermediate file corresponding to the medical record data;
inputting the intermediate file into an image generating model; and
analyzing the intermediate file by the image generating model, and generating and outputting the teeth position chart corresponding to the intermediate file;
wherein the intermediate file has at least one set of teeth position information and at least one set of symptom information respectively corresponding to the at least one set of teeth position information.

2. The method according to claim 1, wherein the step of analyzing the medical record data by the first large language model, and generating and outputting an intermediate file corresponding to the medical record data comprises:
performing a first similarity comparison on the medical record data and a plurality of sets of medical record historical data stored in a historical medical record database, and generating a first similarity comparison result;
retrieving at least one historical medical record from the historical medical record database according to the first similarity comparison result;
performing a second similarity comparison on the medical record data and a plurality of sets of medical record result historical data stored in a historical medical record result database, and generating a second similarity comparison result;
retrieving at least one historical medical record result record from the historical medical record result database according to the second similarity comparison result; and
generating the intermediate file by the first large language model according to the at least one historical medical record and the at least one historical medical record result record.

3. The method according to claim 1, wherein the step of analyzing the medical record data by the first large language model, and generating and outputting an intermediate file corresponding to the medical record data comprises:
performing a third similarity comparison on the medical record data and a plurality of sets of debug data stored in a debug database, and generating a third similarity comparison result;
performing error correction on the medical record data according to the third similarity comparison result, and generating medical record correction data; and
analyzing the medical record correction data by the first large language model, and generating and outputting the intermediate file corresponding to the medical record correction data.

4. The method according to claim 3, wherein the step of analyzing the medical record correction data by the first large language model, and generating and outputting an intermediate file corresponding to the medical record correction data comprises:
inputting the medical record correction data into a second large language model, and determining by the second large language model whether the medical record correction data contains at least one content error;
when the medical record correction data contains the at least one content error, performing error fix on the medical record correction data by the second large language model, until the medical record correction data having undergone the error fix no longer contains the at least one content error; and
when the medical record correction data does not contain the at least one content error, analyzing the medical record correction data or the medical record correction data having undergone the error fix by the first large language model, and generating and outputting the intermediate file corresponding to the medical record correction data or the medical record correction data having undergone the error fix.

5. The method according to claim 1, further comprising:
comparing the intermediate file with a plurality of sets of medical record historical data in a historical medical record database, and determining whether the intermediate file contains at least one set of medical record new data different from the plurality of sets of medical record historical data;
comparing the intermediate file with a plurality of sets of medical record result historical data in a historical medical record result database, and determining whether the intermediate file contains at least one set of medical record result new data different from the plurality of sets of medical record result historical data;
comparing the intermediate file with a plurality of sets of debug data in a debug database, and determining whether the intermediate file contains at least one set of debug new data different from the plurality of sets of debug data; and
when the intermediate file contains at least one of the at least one set of medical record new data, the at least one set of medical record result new data and the at least one set of debug new data, inputting the at least one of the at least one set of medical record new data, the at least one set of medical record result new data and the at least one set of debug new data into the first large language model, and requesting the first large language model to again analyze the medical record data and to again generate and output the intermediate file, until the intermediate file again generated no longer contains the at least one set of medical record new data, the at least one set of medical record result new data and the at least one set of debug new data.

6. The method according to claim 1, further comprising:
generating a treatment plan according to the teeth position chart;
wherein the treatment plan has at least one set of teeth treatment position information and at least one treatment suggestion respectively corresponding to the at least one set of teeth treatment position information,
wherein the at least one set of teeth treatment position information is determined respectively according to at least one set of teeth position labeling information in the teeth position chart, and
wherein the at least one treatment suggestion is determined respectively according to at least one set of symptom remark information in the teeth position chart.

7. The method according to claim 6, further comprising:
transmitting the treatment plan to a user terminal;
receiving a rating result from the user terminal; and
storing the treatment plan and the rating result to a treatment record database.

8. A computer device for generating a teeth position chart, comprising:
a storage module, configured to have a computer program product stored therein; and
a processing module, configured to be coupled with the storage module;
wherein once the processing module loads and executes the computer program product, the processing module is operable to perform steps of:
inputting medical record data into a first large language model;
analyzing the medical record data by the first large language model, and generating and outputting an intermediate file corresponding to the medical record data;
inputting the intermediate file into an image generating model; and
analyzing the intermediate file by the image generating model, and generating and outputting a teeth position chart corresponding to the intermediate file;
wherein the intermediate file has at least one set of teeth position information and at least one set of symptom information respectively corresponding to the at least one set of teeth position information.

9. The computer device according to claim 8, wherein the step of analyzing the medical record data by the first large language model, and generating and outputting an intermediate file corresponding to the medical record data comprises:
performing a first similarity comparison on the medical record data and a plurality of sets of medical record historical data stored in a historical medical record database, and generating a first similarity comparison result;
retrieving at least one historical medical record from the historical medical record database according to the first similarity comparison result;
performing a second similarity comparison on the medical record data and a plurality of sets of medical record result historical data stored in a historical medical record result database, and generating a second similarity comparison result;
retrieving at least one historical medical record result record from the historical medical record result database according to the second similarity comparison result; and
generating the intermediate file by the first large language model according to the at least one historical medical record and the at least one historical medical record result record.

10. The computer device according to claim 8, wherein the step of analyzing the medical record data by the first large language model, and generating and outputting an intermediate file corresponding to the medical record data comprises:
performing a third similarity comparison on the medical record data and a plurality of sets of debug data stored in a debug database, and generating a third similarity comparison result;
performing error correction on the medical record data according to the third similarity comparison result, and generating medical record correction data; and
analyzing the medical record correction data by the first large language model, and generating and outputting the intermediate file corresponding to the medical record correction data.

11. The computer device according to claim 10, wherein the step of analyzing the medical record correction data by the first large language model, and generating and outputting an intermediate file corresponding to the medical record correction data comprises:
inputting the medical record correction data into a second large language model, and determining by the second large language model whether the medical record correction data contains at least one content error;
when the medical record correction data contains the at least one content error, performing error fix on the medical record correction data by the second large language model, until the medical record correction data having undergone the error fix no longer contains the at least one content error; and
when the medical record correction data does not contain the at least one content error, analyzing the medical record correction data or the medical record correction data having undergone the error fix by the first large language model, and generating and outputting the intermediate file corresponding to the medical record correction data or the medical record correction data having undergone the error fix.

12. The computer device according to claim 8, wherein the processing module is further operable to perform steps of:
comparing the intermediate file with a plurality of sets of medical record historical data in a historical medical record database, and determining whether the intermediate file contains at least one set of medical record new data different from the plurality of sets of medical record historical data;
comparing the intermediate file with a plurality of sets of medical record result historical data in a historical medical record result database, and determining whether the intermediate file contains at least one set of medical record result new data different from the plurality of sets of medical record result historical data;
comparing the intermediate file with a plurality of sets of debug data in a debug database, and determining whether the intermediate file contains at least one set of debug new data different from the plurality of sets of debug data; and
when the intermediate file contains at least one of the at least one set of medical record new data, the at least one set of medical record result new data and the at least one set of debug new data, inputting the at least one of the at least one set of medical record new data, the at least one set of medical record result new data and the at least one set of debug new data into the first large language model, and requesting the first large language model to again analyze the medical record data and to again generate and output the intermediate file, until the intermediate file again generated no longer contains the at least one set of medical record new data, the at least one set of medical record result new data and the at least one set of debug new data.

13. The computer device according to claim 8, wherein the processing module is further operable to perform steps of:
generating a treatment plan according to the teeth position chart;
wherein the treatment plan has at least one set of teeth treatment position information and at least one treatment suggestion respectively corresponding to the at least one set of teeth treatment position information,
wherein the at least one set of teeth treatment position information is determined respectively according to at least one set of teeth position labeling information in the teeth position chart, and
wherein the at least one treatment suggestion is determined respectively according to at least one set of symptom remark information in the teeth position chart.

14. The computer device according to claim 13, wherein the processing module is further operable to perform steps of:
transmitting the treatment plan to a user terminal;
receiving a rating result from the user terminal; and
storing the treatment plan and the rating result to a treatment record database.

15. A non-transitory computer-readable recording medium for generating a teeth position chart, once a computer device loads and executes a computer program product stored in the non-transitory computer-readable recording medium, the computer device operable to perform steps of:
inputting medical record data into a first large language model;
analyzing the medical record data by the first large language model, and generating and outputting an intermediate file corresponding to the medical record data;
inputting the intermediate file into an image generating model; and
analyzing the intermediate file by the image generating model, and generating and outputting a teeth position chart corresponding to the intermediate file;
wherein the intermediate file has at least one set of teeth position information and at least one set of symptom information respectively corresponding to the at least one set of teeth position information.

16. The non-transitory computer-readable recording medium according to claim 15, wherein the step of analyzing the medical record data by the first large language model, and generating and outputting an intermediate file corresponding to the medical record data comprises:
performing a first similarity comparison on the medical record data and a plurality of sets of medical record historical data stored in a historical medical record database, and generating a first similarity comparison result;
retrieving at least one historical medical record from the historical medical record database according to the first similarity comparison result;
performing a second similarity comparison on the medical record data and a plurality of sets of medical record result historical data stored in a historical medical record result database, and generating a second similarity comparison result;
retrieving at least one historical medical record result record from the historical medical record result database according to the second similarity comparison result; and
generating the intermediate file by the first large language model according to the at least one historical medical record and the at least one historical medical record result record.

17. The non-transitory computer-readable recording medium according to claim 15, wherein the step of analyzing the medical record data by the first large language model, and generating and outputting an intermediate file corresponding to the medical record data comprises:
performing a third similarity comparison on the medical record data and a plurality of sets of debug data stored in a debug database, and generating a third similarity comparison result;
performing error correction on the medical record data according to the third similarity comparison result, and generating medical record correction data; and
analyzing the medical record correction data by the first large language model, and generating and outputting the intermediate file corresponding to the medical record correction data.

18. The non-transitory computer-readable recording medium according to claim 17, wherein the step of analyzing the medical record correction data by the first large language model, and generating and outputting an intermediate file corresponding to the medical record correction data comprises:
inputting the medical record correction data into a second large language model, and determining by the second large language model whether the medical record correction data contains at least one content error;
when the medical record correction data contains the at least one content error, performing error fix on the medical record correction data by the second large language model, until the medical record correction data having undergone the error fix no longer contains the at least one content error; and
when the medical record correction data does not contain the at least one content error, analyzing the medical record correction data or the medical record correction data having undergone the error fix by the first large language model, and generating and outputting the intermediate file corresponding to the medical record correction data or the medical record correction data having undergone the error fix.

19. The non-transitory computer-readable recording medium according to claim 15, wherein the computer device is operable to further perform steps of:
comparing the intermediate file with a plurality of sets of medical record historical data in a historical medical record database, and determining whether the intermediate file contains at least one set of medical record new data different from the plurality of sets of medical record historical data;
comparing the intermediate file with a plurality of sets of medical record result historical data in a historical medical record result database, and determining whether the intermediate file contains at least one set of medical record result new data different from the plurality of sets of medical record result historical data;
comparing the intermediate file with a plurality of sets of debug data in a debug database, and determining whether the intermediate file contains at least one set of debug new data different from the plurality of sets of debug data; and
when the intermediate file contains at least one of the at least one set of medical record new data, the at least one set of medical record result new data and the at least one set of debug new data, inputting the at least one of the at least one set of medical record new data, the at least one set of medical record result new data and the at least one set of debug new data into the first large language model, and requesting the first large language model to again analyze the medical record data and to again generate and output the intermediate file, until the intermediate file again generated no longer contains the at least one set of medical record new data, the at least one set of medical record result new data and the at least one set of debug new data.

20. The non-transitory computer-readable recording medium according to claim 15, wherein the processing module is operable to further perform steps of:
generating a treatment plan according to the teeth position chart;
wherein the treatment plan has at least one set of teeth treatment position information and at least one treatment suggestion respectively corresponding to the at least one set of teeth treatment position information,
wherein the at least one set of teeth treatment position information is determined respectively according to at least one set of teeth position labeling information in the teeth position chart, and
wherein the at least one treatment suggestion is determined respectively according to at least one set of symptom remark information in the teeth position chart.

21. The non-transitory computer-readable recording medium according to claim 20, wherein the processing module is operable to further perform steps of:
transmitting the treatment plan to a user terminal;
receiving a rating result from the user terminal; and
storing the treatment plan and the rating result to a treatment record database.

22. A method for generating a treatment plan, the method performed when a computer program product is loaded and executed by a computer device, so that the computer device is operable to generate the treatment plan corresponding to medical record data after receiving the medical record data, the method comprising:
inputting the medical record data into a first large language model;
analyzing the medical record data by the first large language model, and generating and outputting an intermediate file corresponding to the medical record data; and
generating the treatment plan according to the intermediate file;
wherein the intermediate file has at least one set of teeth position information and at least one set of symptom information respectively corresponding to the at least one set of teeth position information,
wherein the treatment plan has at least one set of teeth treatment position information and at least one treatment suggestion respectively corresponding to the at least one set of teeth treatment position information,
wherein the at least one set of teeth treatment position information is determined respectively according to the at least one set of teeth position information, and
wherein the at least one treatment suggestion is determined respectively according to the at least one set of symptom information.

23. A computer device for generating a treatment plan, comprising:
a storage module, configured to have a computer program product stored therein; and
a processing module, configured to be coupled with the storage module;
wherein, once the processing module loads and executes the computer program product, the processing module is operable to perform steps of:
inputting medical record data into a first large language model;
analyzing the medical record data by the first large language model, and generating and outputting an intermediate file corresponding to the medical record data; and
generating a treatment plan according to the intermediate file;
wherein the intermediate file has at least one set of teeth position information and at least one set of symptom information respectively corresponding to the at least one set of teeth position information,
wherein the treatment plan has at least one set of teeth treatment position information and at least one treatment suggestion respectively corresponding to the at least one set of teeth treatment position information,
wherein the at least one set of teeth treatment position information is determined respectively according to the at least one set of teeth position information, and
wherein the at least one treatment suggestion is determined respectively according to the at least one set of symptom information.

24. A non-transitory computer-readable recording medium for generating a treatment plan, once a computer device loads and executes a computer program product stored in the non-transitory computer-readable recording medium, the computer device operable to perform steps of:
inputting medical record data into a first large language model;
analyzing the medical record data by the first large language model, and generating and outputting an intermediate file corresponding to the medical record data; and
generating a treatment plan according to the intermediate file;
wherein the intermediate file has at least one set of teeth position information and at least one set of symptom information respectively corresponding to the at least one set of teeth position information,
wherein the treatment plan has at least one set of teeth treatment position information and at least one treatment suggestion respectively corresponding to the at least one set of teeth treatment position information,
wherein the at least one set of teeth treatment position information is determined respectively according to the at least one set of teeth position information, and
wherein the at least one treatment suggestion is determined respectively according to the at least one set of symptom information.
